Europäisches Patentamt

European Patent Office

Office européen des brevets

(19)

(11) **EP 1 424 343 A1**

## (12) EUROPEAN PATENT APPLICATION

(43) Date of publication:
**02.06.2004 Bulletin 2004/23**

(51) Int Cl.⁷: **C07K 14/47**, A61K 49/14,
A61K 47/48, A61P 35/00

(21) Application number: **02026700.1**

(22) Date of filing: **29.11.2002**

(84) Designated Contracting States:
**AT BE BG CH CY CZ DE DK EE ES FI FR GB GR
IE IT LI LU MC NL PT SE SK TR**
Designated Extension States:
**AL LT LV MK RO SI**

(71) Applicant: **Deutsches Krebsforschungszentrum
Stiftung des öffentlichen Rechts
69120 Heidelberg (DE)**

(72) Inventors:
• **Heckl, Stefan
69120 Heidelberg (DE)**
• **Braun, Dr. Klaus
69207 Sandhausen (DE)**

• **Pipkorn, Dr. Rüdiger
69124 Heidelberg (DE)**
• **Waldeck, Waldemar Dr.
69514 Laudenbach (DE)**

(74) Representative: **Schüssler, Andrea, Dr.
Kanzlei Huber & Schüssler
Truderinger Strasse 246
81825 München (DE)**

Remarks:
The sequence listing, which is published as annex
to the application documents, was filed after the date
of filing. The applicant has declared that it does not
include matter which goes beyond the content of the
application as filed.

(54) **Peptide Conjugate useful for cell nucleus molecular imaging and tumor therapy**

(57) Described is a conjugate comprising (a) an amphiphilic transport peptide of human origin as a transmembrane module (TPU), (b) a nuclear localization sequence (NLS) and (c) a signalling and/or drug carrying module (SM), preferably comprising Gd, Ga, Fe, Mn, I and/or F as (diagnostic) image creating compound. Said conjugate is useful for diagnostic purposes, e.g., for cell tracking by MRI, as a contrast agent (e.g., replacing a "biopsy clip") for MRI, or for determining the activity of DNA repair enzymes by MRI. Said conjugate is also useful for therapy, e.g., for chemotherapy or intranuclear Gadolinium Neutron Capture Therapy (GNCT).

The transmembrane module (TPU) is selected among peptides of human origin, whose amino acid sequences are similar to the sequence of the antennapedia fragment RQIKIWFQNRRMKWKK. In a specific embodiment, TPU is derived from the human homeobox protein HOX-B1. The nuclear localization sequence (NLS) is derived from the simian virus 40-T antigen or from a transcription factor.

**EP 1 424 343 A1**

SEQUENCE LISTING

<110> Deutsches Krebsforschungszentrum Stiftung des öffentlichen Rechts

<120> Conjugate useful for cell nucleus molecular imaging and tumor therapy

<130> K 3107EP

<140> EP 02026700.1
<141> 2002-11-29

<160> 17

<170> PatentIn version 3.2

<210> 1
<211> 16
<212> PRT
<213> Artificial

<220>
<223> Transmembrane module

<400> 1

Thr Gln Val Lys Ile Trp Phe Gln Asn Arg Arg Met Lys Gln Lys Lys
1               5                   10                  15


<210> 2
<211> 8
<212> PRT
<213> Artificial

<220>
<223> Nuclear localization sequence

<400> 2

Pro Pro Lys Lys Lys Arg Lys Val
1               5


<210> 3
<211> 7
<212> PRT
<213> Artificial

<220>
<223> Nuclear localization sequence

<400> 3

Pro Lys Lys Lys Arg Lys Val
1               5


<210> 4
<211> 6
<212> PRT
<213> Artificial

<220>
<223> Nuclear localization sequence

<400> 4

Lys Arg Arg Arg Glu Arg
1               5


<210>   5
<211>   7
<212>   PRT
<213>   Artificial

<220>
<223>   Nuclear localization sequence

<400>   5

Lys Ala Arg Lys Arg Leu Lys
1               5


<210>   6
<211>   10
<212>   PRT
<213>   Artificial

<220>
<223>   Nuclear localization sequence

<400>   6

Val Gln Arg Lys Arg Gln Lys Leu Met Pro
1               5                   10


<210>   7
<211>   8
<212>   PRT
<213>   Artificial

<220>
<223>   Nuclear localization sequence

<400>   7

Ser Lys Lys Lys Lys Thr Lys Val
1               5


<210>   8
<211>   8
<212>   PRT
<213>   Artificial

<220>
<223>   Nuclear localization sequence

<400>   8

Gly Arg Lys Arg Lys Lys Arg Thr
1               5


<210>   9
<211>   11
<212>   PRT
<213>   Artificial

<220>
<223>   Nuclear localization sequence

<400> 9

Gly Lys Lys Lys Lys Arg Lys Arg Glu Lys Leu
1               5                   10


<210> 10
<211> 8
<212> PRT
<213> Artificial

<220>
<223> Nuclear localization sequence

<400> 10

Glu Arg Lys Lys Arg Arg Arg Glu
1               5


<210> 11
<211> 7
<212> PRT
<213> Artificial

<220>
<223> Nuclear localization sequence

<400> 11

Phe Lys Lys Phe Arg Lys Phe
1               5


<210> 12
<211> 16
<212> PRT
<213> Artificial

<220>
<223> Antennapedia peptide fragment

<400> 12

Arg Gln Ile Lys Ile Trp Phe Gln Asn Arg Arg Met Lys Trp Lys Lys
1               5                   10                  15


<210> 13
<211> 51
<212> PRT
<213> Artificial

<220>
<223> 1B72:A (of page 6)

<400> 13

Thr Glu Leu Glu Lys Glu Phe His Phe Asn Lys Tyr Leu Ser Arg Ala
1               5                   10                  15


Arg Arg Val Glu Ile Ala Ala Thr Leu Glu Leu Asn Glu Thr Gln Val
            20                  25                  30


Lys Ile Trp Phe Gln Asn Arg Arg Met Lys Gln Lys Lys Arg Glu Arg

35 40 45

Glu Gly Gly
50

<210> 14
<211> 17
<212> PRT
<213> Artificial

<220>
<223> Transport peptide unit

<400> 14

Thr Gln Val Lys Ile Trp Phe Gln Asn Arg Arg Met Lys Gln Lys Lys
1 5 10 15

Cys

<210> 15
<211> 7
<212> PRT
<213> Artificial

<220>
<223> Address peptide

<400> 15

Lys Pro Lys Arg Val Lys Lys
1 5

<210> 16
<211> 15
<212> PRT
<213> Artificial

<220>
<223> Transport peptide unit

<400> 16

Lys Met Thr Arg Gln Thr Trp Trp His Arg Ile Lys His Lys Cys
1 5 10 15

<210> 17
<211> 16
<212> PRT
<213> Artificial

<220>
<223> Transport peptide unit

<400> 17

Gln Met Thr Arg Gln Thr Phe Trp His Arg Ile Lys His Tyr Gly Ala
1 5 10 15

**Description**

The present invention relates to a conjugate comprising (a) an amphiphilic transport peptide of human origin as transmembrane module (TPU), (b) a nuclear localization sequence (NLS) and (c) a signalling and/or drug carrying module (SM). Said conjugate is useful for diagnostic purposes, e.g., for cell tracking by MRI, as a contrast agent (e. g., replacing a "biopsy clip") for MRI, or for determining the activity of DNA repair enzymes by MRI. Said conjugate is also useful for therapy, e.g., for chemotherapy or intranuclear Gadolinium Neutron Capture Therapy (GNCT).

Molecular imaging (MI) is defined as the characterization and measurement of biological processes at the cellular and molecular level (Weissleder and Mahmood, Radiology *219*: 316-333, 2001) and thus necessitates an accumulation of contrast agent within the cells. Until now, however, there were no efficient means for delivering contrast agents into the cell nucleus.

Therefore, it is the object of the present invention to provide a means which overcomes the disadvantages of the tools of the prior art for MI, i.e. which allows to deliver a suitable contrast agent into the cell nucleus.

According to the invention this is achieved by the subject matters defined in the claims. The present invention provides a conjugate comprising (a) a transmembrane module (TPU), (b) a nuclear localization sequence (NLS) and (c) a signalling and/or drug carrying module (SM). During the experiments leading to the present invention, it was realized that the intracellular uptake and cell compartment specificity of the commonly used interstitial contrast agent gadolinium ($Gd^{3+}$) could be helpful for MI. Unfortunately, so far the use of gadolinium ($Gd^{3+}$) contrast agents was limited to the extracellular space. For overcoming this problem, a method was invented with which a delivery of contrast agents like gadolinium into the cytoplasm and finally into the cell nucleus is possible. There have been numerous proposals as to how this could be achieved: Functional peptides such as HIV-1 tat provide a solution for the transport of $Gd^{3+}$ across the cell membranes. The HIV-1 tat peptide has been detected within the cell nucleus. However, there are several signs indicating that HIV-1 tat peptide possesses transactivating properties and can induce apoptosis in hippocampal neurons. The inventors realized that this problem can be overcome by using for the transmembrane transport of $Gd^{3+}$ an amphiphilic transport peptide of human origin (TPU) which, e.g., contains a similar peptide sequence to that of the homeodomain of antennapedia (Derossi et al., J. Biol. Chem. *269*: 10444-10450, 1994. This similar peptide sequence was chosen in order to minimize the risk of immunizing reactions. This transport peptide is part of a modularly constructed CNN-$Gd^{3k}$-complex and is cleavably covalently linked to the nuclear localization sequence (NLS) of SV40T-antigen via a disulfide bond. The NLS is in turn linked to the $Gd^{3+}$-complex via a lysine spacer ($K_2$). After cleavage of the disulfide bond, the NLS becomes the terminal part of the conjugate and can by recognized by the cytoplasmic receptor (importin alfa). After binding of the complex to a second cytoplasmic receptor (importin beta) the entire complex gadolinium-NLS-importin alfa-importin beta is delivered to the nucleus. The nuclear transport uses an active Ran-GDP-system (Gorlich and Mattaj, Science *271*: 1513-1516, 1996). This principle enables the rapid nuclear accumulation of $Gd^{3+}$-complex by means of a Cell Nucleus directed NLS-conjugated $Gd^{3+}$-complex (CNN-$Gd^{3+}$-complex). By use of magnetic resonance imaging, $Gd^{3+}$ was detected within DU-145 prostate cancer cells after only 10 min. The nuclear localization was confirmed with confocal laser scanning microscopy. The resulting MRI signal enhancement only slightly decreased over the next 48 h compared with an absolute loss of signal enhancement after only 8 h when a random target sequence was used.

**Brief description of the drawings**

Figure 1

Left: Spatial representation of one possible configuration of the Bioshuttle conjugate molecule with attached $Gd^{3+}$-complex. The methods used to derive this model are described in detail in Example 1. The transport peptide unit (TPU)(light brown) and the address peptide (AP or NLS)(light blue) are given as ribbon representation of the peptide backbone. The heavy atoms of the Cys-Cys bridge between the two peptide units and the two Lys-residues connecting the $Gd^{3+}$-complex to the NLS are displayed in a 'ball and stick' representation using an atom color code (carbon: green, oxygen: red, nitrogen: blue). The van der Waals spheres of the $Gd^{3+}$-Ion are shown in magenta. Additionally, the hydrogen atoms (white) of the two $H_2O$ molecules in complex with the $Gd^{3+}$ are displayed.

Right: A hydrophobicity color code has been mapped onto the water accessible surface of the conjugate molecule (blue for hydrophilic areas, red for lipophilic areas). Both representations were generated using the InsightII software package. Since no hydrophobicity parameters for the $Gd^{3+}$ complex are available, the surface of this part of the conjugate molecule has been set to white.

Figure 2: HPLC of CNN-Gd$^{3+}$ and CN*R*N-Gd$^{3+}$. Details of the process are described in Example 1

(A) CNN-Gd$^{3+}$; Substance purity: 94.9% according to the HPLC, retention time: 11.9 min.
(B) CNRN-Gd$^{3+}$; Substance purity: 94.9% according to the HPLC, retention time: 13.2 min.

Figure 3: Example of a mass spectrum for a CNN-Gd$^{3+}$ sample

Figure 4

Upper part: Graph of MR signal intensity versus time after incubation with the Gd$^{3+}$ [DTPH]$_4$-HN-K-K-NLS-C-S⌒S-C-TPU ('CNN-Gd$^{3+}$') (red dashed, circles) and the Gd$^{3+}$[DTPH]$_4$-HN-K-K-Random-C-S⌒S-C-TPU ('CNRN-Gd$^{3+}$') (blue dashed, squares) for DU-145 human prostate cancer cells. Data represent three independent experiments.
Lower part: Axial T1-weighted MR images of the cell pellets consisting of $40 \times 10^6$ cells. MEM was used as cell culture medium.

Figure 5

(a) CLSM optical section of living DU-145 prostate cancer cells incubated for 30 min with the modular transport peptide Gd$^{3+}$[DTPH]$_4$-HN-K$^{FITC}$-K-**NLS**-C-S⌒S-C-**TPU**$^{ALEXA}$ conjugate consisting of the human TPU and a Gd$^{3+}$ [DTPH]$_4$-NLS conjugated with a cleavable disulfide linker (100 pM). The green fluorescence signal reveals a distinct nuclear localization of the peptide.

(b) A Gd$^{3+}$[DTPH]$_4$-HN-K$^{FITC}$-K-**Random**-C-S⌒S-C-**TPU**$^{ALEXA}$ conjugate consisting of the human transmembrane carrier TPU covalently attached to a FITC-labeled peptide random sequence was added to the culture medium 30 min prior to CLSM analysis of living DU-145 cells at a concentration of 100 pM. Fluorescence signals were detected exclusively within the cytoplasm, whereas the nuclei remained unstained.

Accordingly, the present invention provides a conjugate comprising (a) an amphiphilic transport peptide of human origin as transmembrane module (TPU), (b) a nuclear localization sequence (NLS) and (c) a signalling and/or drug carrying module (SM), preferably having Gd, Ga, I, Fe, Mn and/or F as image creating compound.
Methods for preparing the components of the conjugates of the present invention and for coupling are, e.g., disclosed in the German Patent Application No. 199 33 492.7. The transport mediator for the cell membrane (= transmembrane module (TPU)) is an amphiphilic transport peptide, preferably of human origin, which can penetrate the plasma membrane. The length of this peptide is not subject to any limitation as long as it has the above property. The cell nucleus addressed delivery system of the present invention is based on the cell immanent Ran/Karyopherine systeme. TPUs suitable for the conjugate of the present invention can be selected according to the methods described in Example 1, e.g., by searching for peptides of human origin containing sequence homologies to the sequence of the Antennapedia peptide fragment RQIKIWFQNRRMKWKK and analysing their capability to pass the cell membrane according to the methods described in Example 1. Examples of TPUs are derived preferably from the penetratin family (Derossi et al., Trends Cell Biol. 8: 84-87, 1998) or are transportan or parts thereof (Pooga et al., The Faseb Journal 12: 68, 1998). Particularly preferred examples of TPUs are derived from Penetratin 1, Antennapedia[1] [Hom][HoxB$_5$], TP[(1AOP/E.Coli)], or PTD[TAT/HIV1]. Further suitable TPUs are HBX5, HBX7 and HXD9. In a preferred embodiment, the transmembrane module (TPU) is the human homeobox protein HOX-B1 or a fragment or derivative thereof having the same biological activity, i.e. can still pass the cell membrane.
The term "derivative" in this context means that the amino acid sequences of these molecules differ from the sequences of the original molecule (HOX-B1) (due to substitution(s), addition(s) and/or deletion(s) of one or more amino acids) at one or several positions but have a high level of identity to these sequences. Identity hereby means an amino acid sequence identity of at least 60 %, in particular an identity of at least 80 %, preferably of more than 90 % and particularly preferred of more than 95 %.
In a particularly preferred embodiment, the transmembrane module (TPU) comprises the amino acid sequence TQVKIWFQNRRMKQKK.
The transmembrane module (TPU) is produced biologically (purification of natural transmembrane peptides or fragments thereof, or cloning and expression of the sequence in a eukaryotic or prokaryotic expression system), preferably synthetically, e.g., according to the well established "Merrifield method" (Merrifield, J. Am. Chem. Soc. 85: 2149, 1963).

Suitable nuclear localization sequences (NLS) are known to the person skilled in the art. Examples of suitable NLS are: (a) -Pro-Pro-Lys-Lys-Lys-Arg-Lys-Val and (b) $H_3N^+$-Pro-Lys-Lys-Lys-Arg-Lys-Val- (from SV40-T-Antigen; see Kalderon et al., Cell 39: 499-509, 1984). Further examples are -Lys-Arg-Arg-Arg-Glu-Arg- (= KRRRER) and -Lys-Ala-Arg-Lys-Arg-Leu-Lys- (=KARKRLK), for simian CMV which resembles the simian virus 40 large-T-antigen NLS (J. Viol. 1998, 72(10): 7722-7732). Further suitable are NLS from transcription factors:

| NF-kappaB | VQRKRQKLMP |
|---|---|
| TFIIE-β | SKKKKTKV |
| Oct-6 | GRKRKKRT |
| TCF-1-alpha | GKKKKRKREKL |
| HATF-3 | ERKKRRRE |
| C. elegans SDC$_3$ | FKKFRKF |

The signalling and/or drug carrying module is not subject to limitations. It can be chosen freely, depending on the effect which shall be produced in the cell. The nature of the signalling module depends on the desired diagnostic method which might be based on optical methods, SPECT, PET, gamma-camera, MRI (magnetic resonance imaging, synonym: MRT = magnetic resonance tomography) or CT(computer tomography). The person skilled in the art knows such (diagnostic) image creating compounds. Preferably, the signalling and/or drug carrying module (SM) comprises Gd, Ga, Mn, Fe, I and/or F, most preferably Gd, as such image creating compound. Preferably, said atoms or ions are linked to the nuclear localization sequence as a chelate complex using, e.g., as the chelating agent diethylenetriaminepentaacetic acid (DTPA), Gd-BOPTA, Gd-DOTA, GD-EOB-DTPA, Gd-DTPA-BMA, Gd-HP-DOBA, Gd-DTPA-BMEA-Gd-HIDA, Mn-DPDP or "cyclized DTPA" (wich is particularly suitable in view of its physico-chemical properties), as described in the Examples below.

The conjugate of the present invention, preferably contains (a) spacer(s) which is (are) preferably located between the transmembrane module (TPU) and the nuclear localization sequence (NLS) and/or NLS and the signalling and/or drug carrying module (SM). The spacer serves for eliminating or positively influencing optionally existing steric hindrances between the modules and/or allows to separate modules from each other, e.g., in the cytoplasma of a cell.

In a preferred embodiment, the transmembrane module (TPU) of the conjugate of the present invention is coupled to the NLS via a covalently cleavable spacer I and/or the NLS is coupled to the signalling and/or drug carrying module (SM) or a compound trapping the signalling and/or drug carrying module (SM) via a covalently non-cleavable spacer II.

In a more preferred embodiment, spacer I comprises a redox cleavage site, e.g. a disulfide bridge (-cysteine-S-S-cysteine-O-N-H-). The binding formed between the transmembrane module (TPU) and the NLS is a redox coupling (mild cell-immanent bond by means of DMSO; Rietsch and Beckwith, 1988, Ann. Rev. Gent 32: 163-184):

Cysteine-SH     SH-cysteine     ⇒     cystine-S-S-cystine

The coupling of the constituents thereto is made by covalent chemical binding. The redox cleavage site is inserted chemically between TPU and NLS by the above mentioned redox coupling. There is also a covalent bond, preferably an acid amide bond, between the optionally present spacer(s) and the module(s) of the conjugate. Possible alternatives are ether or ester bonds, depending on the functional group(s) present in the substance to be conjugated.

In an even more preferred embodiment, spacer II of the conjugate of the present invention is polylysine, polyglycine or poly(glycine/lysine). The length of spacer II, preferably, is two to six amino acids. In a particularly preferred embodiment, spacer II is -glycine-glycine- (G$_2$).

The nuclear localization sequence (NLS), signalling and/or drug carrying module (SM) and/or spacer II may optionally be labelled, e.g., radioactively, with a dye, with biotin/avidin, etc. Preferably, spacer II carries an FITC-label.

The most preferred embodiment of the conjugate of the present invention has the following structure: transmembrane module (TPU) - spacer I comprising a cleavable disulfide bridge - nuclear localization sequence (NLS) - spacer II - signalling and/or drug carrying module (SM) or compound trapping the signalling and/or drug carrying module + signalling and/or drug carrying module (SM).

The present invention also relates to various diagnostic and therapeutic uses of the conjugates of the invention.

Accordingly, the present invention relates to the use of the above conjugate for the preparation of a diagnostic composition for cell tracking. So far, for monitoring the proliferation of particular cells (tumor cells, lymphocytes, oligodendrocytes, macrophages) within an organism via MRI, the cells had to be incubated outside the organism with MION (micro oxide (Fe$_2$O$_3$) nanoparticles) which were transported into the cells by transferrin receptors located at the cell surface. However, the efficiency of delivery varies depending on the type of cell, since, e.g., tumor cells show a higher expression of transferrin receptors compared to normal cells (e.g. lymphocytes). Moreover, only limited amounts of iron are taken up by cells, since cells protect themselves from iron intoxication. So far, the problem of the low number of transferrin receptors on the surfaces of normal cells and the inefficient delivery of MIONs has been tried to overcome by coupling the MIONs to the transport protein *HIV-tat* in each cell (lymphocytes and tumor cells). However, this approach was not very promising due to the rapid lowering of the signal intensity of the T2-weighted MRT image (within some days). Moreover, the use of *HIV*-tat for intracellular accumulation of MIONs, gadolinium etc. is problematic, since *HIV*-tat (which is part of the AIDS-virus) is transactivating and can induce damages of the hippocampus.

"Tumor" in the context of the present application means any neoplastic cell growth. They include cancer of the bladder, bone, bone marrow, brain, breast, cervix, gall bladder, gastrointestinal tract, kidney, liver, lung, muscle, ovary, pancreas, parathyroid, prostate, skin, salvary glands, spleen, testis, uterus, particularly brain.

For cell tracking via MRI, compounds have to be used which do not interfere with the growth of cells, e.g., tumor cells since this would result in a misinterpretation of the effect of the drug used for chemotherapy.

The effect of chemotherapy or viral therapy in neurooncology can be studied, e.g., by use of rat glioma. For doing so, about $10^5$ glioma cells are implanted into a rat brain, after about ten days a tumor is formed. For evaluating the effect of a therapy, the size of the tumor is monitored for about four weeks and the results obtained are compared with the results obtained with untreated animals. For each MRI, an interstitial gadolinium (Gd$^{3+}$) preparation has to be administered to the animal via the tail vein. However, due to the fact that this preparation is cleared from the body very rapidly, it has to be newly administered for each MRT. Thus, for a observation period of about 30 days large amounts of contrast agent are needed.

To summarize, the methods for cell tracking used so far show a number of disadvantages. The use of MIONs is limited to T2-weighted sequences since otherwise a lowering of signal intensity can not be achieved. However, this approach leads to susceptibility artifacts. Compared to gadolinium, the signal/noise ratio decreases, i.e., the structures become blurred. Since the expression of the transferrin receptor is autoregulated the cell can accumulate only limited amounts of iron. Thus, the desired lowering of the signal intensity of the T2-weighted sequence is often too low. As regards the T1-weighted sequence, the increase of signal intensity by use of MIONs is negligible. Moreover, the application of MIONs is restricted to cells showing a high concentration of transferrin receptors on their surfaces (in particular, tumor cells showing an increased iron metabolism). Lymphocytes hardly accumulate iron.

These problems associated with the methods of cell tracking of the prior art and disadvantages can be overcome by use of the conjugates of the present invention. When using, e.g., CNN-Gd$^{3+}$ the cells which shall be implanted are incubated with the conjugate prior to implantation. The CNN-Gd$^{3+}$ will be permanently trapped in the nucleus of the tumor cells with high concentration and is evenly distributed to the nuclei of dividing tumor cells during growth of the tumor. Since CNN-Gd$^{3+}$ is localized within the cell and can not leak into the interstice generated by the tumor, the image of the T1-weighted sequence only shows tumor cells (with the edges of the tumor being more clearly compared to the normally used interstitial gadolinium preparations). In case of transplantation of neural stem cells by use of the conjugate of the present invention it is possible to determine regions of the CNS which had been restored.

Oligodendrocytes and precursors thereof can be visualized after incubation with a conjugate of the present invention, e.g., CNN-Gd$^{3+}$, in the T1-weighted MRT sequences for monitoring (a) their capability for migration and (b) the success of a remyenilisation therapy in case of myelin deficient rats. So far, for this approach MIONs had been used. Since, however, the use of MIONs is restricted to T2-weighted sequences (signal/noise ratio increased) and associated with susceptibility artifacts, the use of the conjugates of the present invention is advantageous.

Incubation of lymphocytes with the conjugate of the present invention, e.g., CNN-Gd$^{3+}$, outside of the organism (*ex vivo*) and subsequent intravenous injection leads to tumor edges showing increased signal intensity in the T1-weighted sequences (low signal/noise ratio) since the lymphocytes migrate to (and remain at) the edges of the tumor. Since CNN-Gd$^{3+}$ is intracellularly localized within the lymphocytes it can be expected that - in contrast to the interstitial Gd-preparations used so far - it will not leak along the interstice (blurred tumor edges). Incubation of lymphocytes with the conjugate of the present invention allows to show the beta-islets of the pancreas of rates (type I diabetes) even in the T1-weighted sequences (low signal/noise ratio).

To summarize, cell tracking using the conjugates of the present invention offers many advantages.

Due to the fact that the delivery of the conjugate does not depend on the level of transferrin receptors on the cell surface migration of non-tumor cells (e.g. stem cells) can be monitored. Since the conjugate of the present invention does not exhibit transactivating characteristics it does not interfere with tumor growth, thus is useful in trials for studying the effects of chemotherapeutics. The use of the conjugate of the present invention is cost and time saving since a single injection with a low dose (e.g. 0.5 mM) is sufficient. If tumor cells that shall be implanted had been incubated with the conjugate prior to implantation, they can be immediately localized in the brain of a rat in the T1-weighted sequence. Since the conjugate accumulates in the nucleus and cannot leak into the interstitial space generated by the tumor a T1-weighted image can be obtained, showing exclusively tumor cells (with the tumor edges being sharper compared to the use of the normal Gd-preparation). Susceptibility artifacts and the limitation to T2-weighted sequences (increased noise/signal ratio) can be avoided.

The present invention also relates to the use of a conjugate of the present invention for the preparation of a contrast agent for MRI replacing a biopsy clip.

For biopsy of tumors of the breast or brain, a metal clip is clipped to the site of biopsy allowing to mark this site. The knowledge of the site of biopsy is important for future biopsies or operations. However, the presence of such an alien element within the body is weighing heavily on the minds of the patients. Moreover, the presence of this clip causes artifacts in MRI checkups. The main problem, however, is that during growing of the tumor the clip is shifted to a different site. Thus, regular control imaging examinations are required for monitoring the migration of the clip. This problem has been overcome by use of the conjugate of the present invention (e.g., CNN-Gd$^{3+}$; 0,5 mM) for marking a site of biopsy, e.g., via the biopsy needle. At the site of biopsy some drops of the conjugate are applied. After a few minutes the conjugate accumulates in the cell nucleus and is trapped within the nucleus over a long period of time. The contrast agent will "grow" together with the tumor and will a appear as a "tail" in the T1-weighted MRT image. This "tail" is formed by cells which are exclusively derived from the cells that had been previously marked with the contrast agent. In other word, the site of biopsy is permanently marked and this is independent of the future behavior of the tumor. Regular control imaging examinations are no longer required, since by monitoring the "tail" the original site of biopsy can be determined.

The present invention also relates to the use of the conjugate of the present invention for the preparation of a diagnostic composition for determining the activity of DNA repair enzymes.

The DNA repair enzyme O(6)alkylguanine-DNA-alkyltransferase (AGT) is very important in the therapy of brain tumors using alkylating drugs (like Temodal$^{TM}$, BCNU etc.), since this enzyme is responsible for reversing the alkylation of the guanine base. Actually, the activity of AGT directly correlates with the time of survival after therapy with alkylating drugs. However, the activity of AGT varies from tumor to tumor and patient to patient. Thus, the determination of the activity of AGT in the tumor cells prior to the therapy with alkylating substances is desirable for prognosis. For example, about 25% of patients having a glioma show an almost complete loss of activity of AGT. Such patients have to be selected prior to operation and radiation therapy by MRI, thus allowing to start the efficient therapy with alkylating drugs as soon as possible since radiation treatment performed later on transiently induces AGT. So far, the determination of the acitivity of DNA repair enzymes which might interfere with a planned chemotherapy by MRI is not possible and, for doing so, tumor biopsies have to be taken and the samples are analyzed by immunofluorescence with monoclonal antibodies.

The above problems can be overcome by use of the conjugate of the present invention, e.g., CNN-Gd$^{3+}$, which allows to determine the activity of DNA repair enzymes like AGT in a different way. For the generation of a signal in the T1-weighted MRT-sequence the interaction of one of the nine coordination spheres of the gadolinium complex within the CNN-Gd$^{3+}$ with water protons is required. If all of the coordination spheres are occupied, the contrast agent is inactive and no signal is generated. The 9$^{th}$ coordination sphere can be blocked by a substrate for AGT (inactive contrast agent). Only in case that AGT removes this substrate, water protons can interact with the gadolinium resulting in the generation of a signal in the T1-weighted sequence. Accordingly, the determination of the activity of DNA repair enzymes like AGT in the cell nucleus can be carried by use of CNN-Gd$^{3+}$ conjugate with the 9$^{th}$ coordination sphere being blocked with a substrate for the enzyme.

The conjugate of the present is also useful for therapy of tumors, preferably brain tumors. Examples of therapeutic strategies are described below.

Thus, the present invention also relates to the use of the conjugate of the present invention for the preparation of a pharmaceutical composition for the chemotherapeutical treatment of a tumor. This treatment is based on (a) reducing the level of active DNA repair enzymes (i.e. alkyltransferases) and/or (b) efficiently delivering alkylating compounds into the nucleus of tumor cells.

The efficiency of alkylating drugs for chemotherapy can be enhanced by inactivating the DNA repair enzyme AGT with O(6)-benzylguanine (O(6)benzyl-2'-deoxyguanosine) since AGT not only removes the alkyl residue form the guanine base (at O(6)-position) of the DNA, but, in addition, removes this residue from O(6)-benzylguanine that has been intravenously applied and accumulates in the cell nucleus. This reaction is irreversible, in other words after removal of the alkyl residue from O(6)-benzylguanine AGT is irreversibly inactivated and is rapidly degraded by proteases. As long as the level of active AGT is low (i.e. before sufficient amounts of ATG are newly synthesized within the cell) the tumor cells are sensitized for chemotherapeutical treatment with alkylating substances. Unfortunately, only limited amounts of the i.v. administered alkylating drug arrive at the tumor, e.g. a brain tumor. (For example, only about 30% of Temodal$^{TM}$ of the blood pass the blood-brain barrier). Moreover, it is totally unclear how many percent of intravenously administrated O(6)-benzylguanine (that can pass the blood-brain-barrier) finally arrive in the nuclei of tumor cells, if ever.

The efficiency of the delivery of O(6)-benzylguanine to the tumor cells and, therefore, the efficiency of irreversible inactivation of AGT can be improved by administrating O(6)-benzylguanine bound to the conjugate of the present invention, e.g. by use of O(6)benzylguanine occupying the 9th coordination sphere of the CNN-Gd$^{3+}$ conjugate.

Additionally, this approach can be applied for efficient delivery of a chemotherapeutical drug, e.g. an alkylating compound. The compound is covalently coupled to the conjugate of the present invention, e.g. the CNN-Gd$^{3+}$ conjugate, and, thus, can efficiently the blood-brain-barrier, the cell membrane and the membrane of the nucleus.

Finally, the present invention relates to the use of a conjugate of the present invention for the preparation of a pharmaceutical composition for the intranuclear GNCT-treatment of a tumor.

So far, Gadolinium Neutron Capture Therapy (GNCT) was only applied to cell cultures and animals using interstitial gadolinium preparations. Due to the great distance of the gadolinium from the desired target (DNA, nucleus), therapeutic effects could hardly be observed. Intranuclear GNCT could only be applied (a) to glioblastoma cell cultures and (b) required extremely high concentrations of gadolinium (up to 20 mg/ml) and very long incubation periods (125 hours). Thus, this approach is not suitable for therapy of human patients. According to the present invention, the problems associated with GNCT can also be overcome by using the conjugates of the present invention allowing to deliver substances like gadolinium into the cell nucleus. Thus, by using e.g., the CNN-Gd$^{3+}$ conjugate a very efficient GNCT (in the cell nucleus !!) can be achieved and, moreover, the efficiency of therapy can be monitored by simultaneously carrying out MRT. For this approach only low concentrations of the conjugate (e.g., 0.5 mM gadolinium) are required. Since gadolinium ($^{157}$Gd$^{3+}$) has a high capture range for neutrons (much higher compared to $^{10}$B; $^{157}$Gd: 254,000 barn, $^{10}$B: 3,840 barn) it is particularly suitable for intracellular GNCT: After neutron radiation treatment, irreparable damages of the DNA are induced with the extent of damages depending on the proximity of the gadolinium to the DNA.

The efficiency of a conjugate of the present invention as a pharmaceutical composition can be further increased by coupling a cytotoxic compound to said conjugate. Cytotoxic compounds which may be covalently coupled to the conjugate are preferably Temodal, Dacarbazin, BCNU, methotrexate, cis-platin, etoposide, taxols, etc.

The present invention is explained by the examples.

**Example 1**

**General Methods**

**(A) Identification of Transport Peptide Unit (TPU) structures by 'SRS' Biocomputing**

A FASTA search was carried out in the HUSAR Sequence Retrieval System (SRS). It was searched for peptides of human origin containing sequence homologies to the sequence of the Antennapedia peptide fragment 'RQIKIWFQNRRMKWKK'. Among several domains this Smith-Watermann Score: 1B72:A HOMEOBOX PROTEIN HOX-B1: 86.667% identity (86.667% ungapped) in 15 aa overlap was detected:

```
                                                  10

      pAnt                              RQIKIWFQNRRMKWKK

                                        :.:::::::::: ::

   1B72:A  TELEKEFHFNKYLSRARRVEIAATLELNETQVKIWFQNRRMKQKKREREGG
              50          60          70          80          90
```

In order to find the optimal sequence- and structural homologues the amino acid sequence with the above described score '1B72:A' was selected and was further used as the transport peptide unit (TPU). In the alignment, identical amino acids were displayed with ':' and the similarities with '.'. This sequence was chosen due to a lesser risk of immunizing reactions.

**(B) Synthesis of the CNN-Gd$^{3+}$-complex and the CNRN-Gd$^{3+}$-complex**

The modules and conjugates used in the present study are shown in Table 1

## Table 1

## Biochemical design of the functional modules used in the MRI and CLSM study.

| Prod. No. | module | scheme | sequences |
|---|---|---|---|
| | | **1B72:A HOMEOBOX PROTEIN HOX-B1** | |
| #3723 AC P52751 | Transport-peptide | TPU (human) | H2N-TQVKIWFQNRRMKQKK-(Cys-CO-NH2)-(SH)-CONH** |
| | | **ADDRESS-PEPTIDE** | |
| NLS-[SV40T-antigen] Random | NLS Random | | $H_2$N-PKKKRKV-(Cys-CO-$NH_2$)-(SH)-CONH$_2$** $H_2$N-KPKRVKK-(Cys-CO-$NH_2$)-(SH)-CONH$_2$** |
| | | **MRI** | |
| #3723 #1552a #1552b | TPU NLS Random | 'CNN-Gd$^{3+}$-complex' 'CN$R$N-Gd$^{3+}$-complex' | Gd$^{3+}$[DTPH]$_4$-HN-K$_2$-**NLS**-C-S$^∩$S-C-TPU Gd$^{3+}$[DTPH]$_4$-HN-K$_2$-**Random**-C-S$^∩$S-C-TPU |
| | | **CLSM** | |
| #3723 f #1552 af #1552 bf | $^{ALEXA}$TPU NLS-FITC Random-FITC | 'CNN$^{FITC}$-Gd$^{3+}$-complex' 'CN$R$N$^{FITC}$-Gd$^{3+}$-complex' | Gd$^{3+}$[DTPH]$_4$-HN-K$^{FITC}$-K-**NLS**-C-S$^∩$S-C-TPU$^{ALEXA}$ Gd$^{3+}$[DTPH]$_4$-HN-K$^{FITC}$-K-**Random**-C-S$^∩$S-C-TPU$^{ALEXA}$ |

#1552a: Nuclear Localization-Sequence (CNN) Conjugated-Gd$^{3+}$-Transporter; #1552b: Random-Sequence (CN$R$N)-Conjugated-Gd$^{3+}$-Transporter; #1532af: Nuclear Localization-Sequence (CNN) Conjugated-Gd$^{3+}$-Transporter for CLSM; #1552bf: Random-Sequence (CN$R$N)-Conjugated-Gd$^{3+}$-Transporter for CLSM; S$^∩$S: Cleavable spacer; **: Single letter amino acid code; AC P52751: Accession number SRS data base.

To perform solid phase synthesis of peptide modules the Fmoc-strategy was used in a fully automated synthesizer Syro II (MultiSyn Tech, Germany) described by Merrifield (J. Am. Chem. Soc. *85*:2149-2154 (1963))). The syntheses of TPU transmembrane peptide (#3723; Table 1), the NLS CNN-Gd$^{3+}$-complex (Table 1, #1552a) and the random NLS CNRN- Gd$^{3+}$-complex (Table 1, #1552b) were performed with an identical procedure. Stochiometric amounts of NLS-K$_2$-DTPH-peptide and Gd$^{3+}$ (Sigma-Aldrich, Germany; Cat. No. G7532) were solved in an aqueous NaCl-solution (0,9%). After 12 hours the complexation process was stopped. The complex-formation of the Random-K$_2$-DTPH-peptide and Gd$^{3+}$ was performed with an identical procedure.

Both the modular $^{ALEXA}$CNN$^{FITC}$-Gd$^{3+}$-Complex (Table 1, #1552af) and the $^{ALEXA}$CN$R$N$^{FITC}$- Gd$^{3+}$-complex (Table 1, #1552bf) are composed of an ALEXA®546 (Molecular Probe, Eugene, OR, USA). Fluor-tagged cellular membrane transport peptide (TPU) and a FITC-tagged Gd$^{3+}$-complex covalently linked to a nuclear localization sequence (NLS [SV40-T]). A cysteine-mediated disulfide bond enables the cleavable connection between the TPU and the NLS.

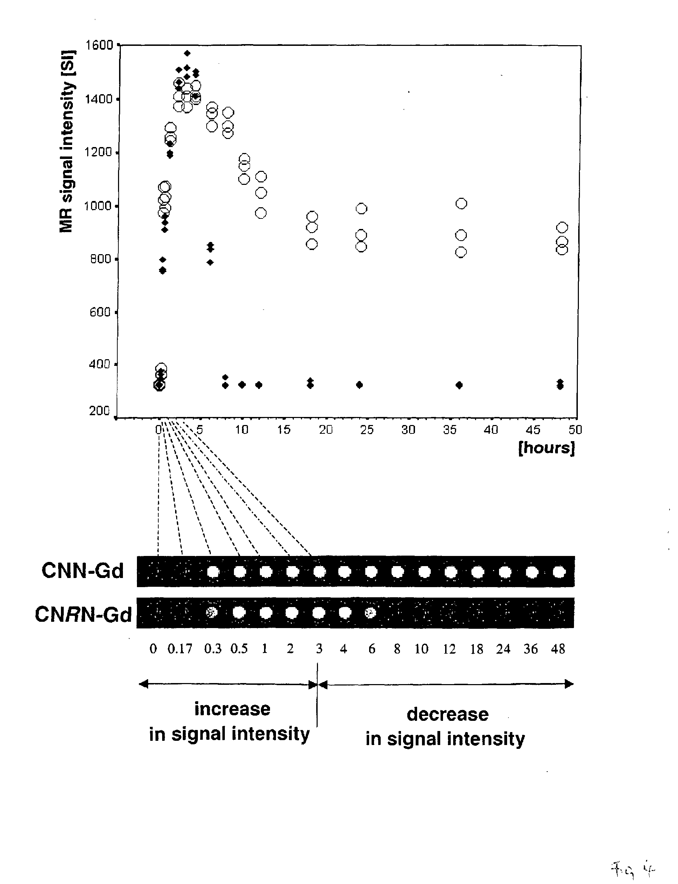

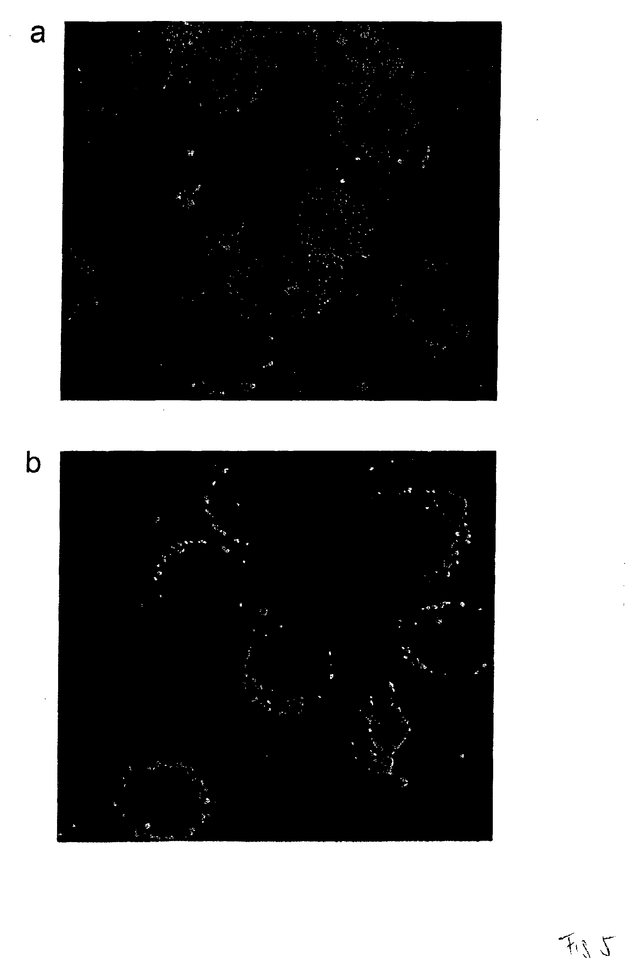

All products were precipitated in ether and purified by preparative HPLC (Shimazu LC-8A, Shimadzu, Duisburg, Germany)) on a YMC ODS-A 7A S-7 µm reverse phase column (20 × 250 mm) using 0.1% trifluoroacetic acid in water (A) and 60% acetonitrile in water (B) as eluent. Peptides were eluted with a successive linear gradient increasing from 25% to 60% B-eluent in 49 min at a flow rate of 10 ml × min$^{-1}$. The fractions corresponding to the purified conjugate were lyophilized. Sequences of single modules as well as the complete bimodular construct were characterized with analytical HPLC (Shimadzu LC-10, Japan) using a YMC-Pack Pro C18 (150×4.6 mm ID) S-5µm, 120A-column with 0.1% trifluoracetic acid in water (A) and 20% acetonitrile in water (B) as eluent. The analytical gradient ranged from 5% (B) to 80% (B) in 35 minutes (Figure 4). Further characterization was performed with laser desorption mass spectrometry (Finnigan, Vision 2000, , Thermguest Analyt. Systeme, Egelsbach, Germany)) (Figure 5). Cysteine groups of the human transmembrane peptide TPU [$H_2N$-TQVKIWFQNRRMKQKK-(Cys-CO-$NH_2$)-(SH)-$CONH_2$] (Table 1, #3723) and the NLS peptide module $Gd^{3+}$-compound ($Gd^{3+}$-[DTPH]$_4$-$K_2$-[PKKKRKV]-HN-Cys-(SH)-$CONH_2$, (NLS$_{SV40T}$)} (Table 1, #1552a) were oxidized at the range of 2 mg $\times$ ml$^{-1}$ in a 20% DMSO water solution. 5 hours later the reaction was completed. The random sequence peptide (Table 1, #1552b) was linked under identical conditions. The progress of oxidation was monitored by analytical C18 reverse phase HPLC.

### (C) Cell culture

Cell culturing of DU-145 prostate cancer cells was performed as described previously (Braun et al., J. Mol. Biol. *318*:237-243, 2002).

### (D) Localization of the NLS-$Gd^{3+}$-Complex by CLSM

To perform fluorescence microscopic studies, DU-145 cells and lymphocytes ($5 \times 10^5$) were incubated for 24 hours in quadriperm® plus (Heraeus, Germany; Cat. No.: 76077310) containing sterile glass coverslips. After two wash-cycles with MEM, the cells were incubated with ALEXA CNN$^{FITC}$-$Gd^{3+}$-Complex (Table 1, #1552af) and ALEXACN*RN*$^{FITC}$-$Gd^{3+}$-complex (Table 1, #1552bf) (100 pM) at 37°C in a 5% $CO_2$ atmosphere for 30 min. The culture medium was removed to enable later microscopic studies. The cells were washed twice and were finally embedded in Moviol®. The verification of the intracellular distribution of the ALEXA® 546 Fluor and FITC bi-labeled ALEXACNN$^{FITC}$-$Gd^{3+}$-complex and ALEXACN*RN*$^{FITC}$-$Gd^{3+}$-complex in living DU-145 cells and lymphocytes was conducted by a Zeiss laser confocal microscope (LSM 510 UV). For excitation of the FITC a filter set with 488 nm and 522 nm emission filter was used. In case of ALEXA® 546 Fluor, excitation was achieved by a 543 nm filter with subsequent filtering of the emission by a 580 nm filter. The optical slice thickness was 700 nm. The excitation line of an argon/krypton laser was used to detect fluorescence signal from ALEXACNN$^{FITC}$-$Gd^{3+}$-complex (Table 1, #1552af) and ALEXACNRN$^{FITC}$-$Gd^{3+}$-complex (Table 1, #1552bf). To increase the contrast of optical sections, 12-20 single exposures were averaged. Parameters of the image acquisition were adapted to show signal intensities in accordance with the visual microscopic image.

### (E) Measurement of nuclear $Gd^{3+}$ concentration using Inductively coupled Plasma Mass Spectrometry (ICP-MS)

The nuclear uptake of CNN-$Gd^{3+}$ was examined by a mass spectrometrical method: Gd concentration measurements were carried out by means of a high resolution element mass spectrometer (Finnigan MAT ELEMENT2, Bremen, Germany) with inductively coupled plasma (ICP-MS) at a resolution ($\Delta$m $\times$ m$^{-1}$) of 4000. The instrument was equipped with a self aspirating 100μl $\times$ min$^{-1}$ PFA-Nebulizer and spray chamber, standard injector and torch. Instrument and operation parameters were as follows: plasma power = 1100 W, cool gas flow = 15.5 L $\times$ min$^{-1}$, auxiliary gas flow = 1 L $\times$ min$^{-1}$, sample gas flow = 1 L $\times$ min$^{-1}$, mass window = 850%, search window =800%, integration window = 80%, samples per peak = 30, No. of scans = 3. Internal standard correction and drift correction were active for $^{103}$Rh (for ICP, Merck, Germany, diluted to 5 ng $\times$ ml$^{-1}$). Before measurement the instrument was tuned and calibrated using 1 ng $\times$ ml$^{-1}$ multi-element standard solution (Merck, Germany).

### (F) Sample preparartion

All preparations of samples and standards were carried out at clean flow box benches. For dilutions, purified and background measured water was used (> 17.5 M$\Omega$ $\times$ cm$^{-1}$).
A closed, pressurized microwave digestion unit (Mars5, CEM GmbH, Germany) equipped with a rotor for 14 vessels, each containing three Teflon® vessels with a volume of 3 ml was used for digestion of samples containing cellular components. Aliquots of 200 μl sample (cells and supernatant) and 200 μl $HNO_3$ 60% (grade: Ultra pur®, Merck, Germany) were digested as follows: Ramp of 15 min, 300 W, 100%, 0.5 bar, 150°C, holding for 10 min, ramp of 15 min, 300 W, 100%, 0 bar to cool to room temperature, holding for another 5 min. The samples were diluted by additional 600 μl $HNO_3$. Before measurement 200 μl of the samples were diluted by 700 μl $H_2O$ and 100 μl $^{103}$RhCl$_3$ standard solution (E. Merck, Darmstadt, Germany) was added. 200 μl of cell free suspensions were diluted with 600 μl $H_2O$ and 100 μl of $HNO_3$ and 100 μl of $^{103}$Rh standard solution were added. In accordance with the above-mentioned pretreatment, the samples (supernatant and cell pellet) were each measured separately. With the resulting data, a standard curve was formulated from several differing $Gd^{3+}$ concentrations. This standard curve along with a second control curve (only clear water with no chemical ingredients) served for a comparable evaluation (ICP-MS).

**(G) Cell viability**

Cell viability was assessed by dye exclusion assay. DU-145 cells were incubated with both conjugates (CNN-Gd$^{3+}$-complex and CN$R$N-Gd$^{3+}$-complex) (0,5 mM) for 24, 48 and 72 hours. Non-treated cells served as controls for the same time periods. Five minutes after trypan blue staining (0.4%), viable and nonviable cells were microscopically quantified. Cell counts for each experimental series were repeated twice.

**(H) Molecular Modeling**

Because no experimental data for these modules was available, spatial models were generated based on homologous data. The objective of the following spatial model is a representation of the relative magnitudes of the component units and an approximation of their respective structures for the purposes of visualization and is, as such, not exactly representative of the molecular structure. The spatial model of the bioconjugate was formed by manual connection of the molecular modules (TPU, NLS, DPTH). The FASTA search option of the Protein Data Bank (Berman et al., Nucleic Acids Res. 2:235-242, 2000) was used to identify sequences which show high similarity with the TPU (KMTRQTWWHRIKHKC) and the NLS-Sequence [PKKKRKV]. In the case of TPU, the crystal structure of the site-specific recombinase, XerD (PDB 1A0P :217-231 sequence QMTRQTFWHRIKHYA) was taken as a template for which an 85% identity in 13 amino acids overlap was shown. For NLS, a part of the crystal structure of the tissue transglutaminase (PDB-entry 1KV3: 598-605: PKQKRKLV) was taken which in turn showed an 71% identity in seven amino acids overlap. Although the sequences are too short to provide highly reliable spatial structures, this approach seems to be justified to generate models for the purpose of visualization. The biopolymer option of the INSIGHTII module was applied to mutate the required amino acids. A minimization using the AMBER-Force field was followed by a short molecular dynamics simulation in aqueous solution to relax the constructed model. The Gd$^{3+}$-complex was taken from the Cambridge Structural Database (entry heqbua) (Allen and Kennard, Chemical Design Automation News, 31-37, 1993). The aforementioned molecular modules were connected using INSIGHTII software (Accelrys, 9685, Scranton Road, San Diego, CA 92121-2777). INSIGHTII was also used to produce the graphical representations of the bioconjugates.

**(I) MRI: DU-145 Cell uptake of the CNN-Gd$^{3+}$-complex and CN$R$N-Gd$^{3+}$-complex compared to that of Magnevist®**

DU-145 cells were harvested and divided into tubes (Falcon®, Becton Dickinson USA, Cat. No.: 35.2096) (Cell No.: 40 $\times$ 10$^6$ cells per tube). The CNN-Gd$^{3+}$-complex (Table 1, #1552a), the CNRN-Gd$^{3+}$-complex (Table 1, #1552b) and the Magnevist® were each dissolved in MEM in a concentration of 0.5 mM and were then incubated for 10, 20, 30 minutes up to 3 hours. After centrifugation of the tubes (800 rpm x 10 min), the incubation medium (supernatant) was removed and the cells (pellet) were washed twice with MEM without conjugates to remove all unbound Gd$^{3+}$-DTPH (Magnevist®), CNN-Gd$^{3+}$-complex (Table 1, #1552a) and CNRN-Gd$^{3+}$-complex (Table 1, #1552b).
MR imaging used a 1.5-T whole body Siemens Magnetom Vision Plus with a standard circular polarized head coil. The test tubes were firmly positioned parallel to each other totally submerged in a water bath. The imaging protocol consisted of an axial T1-weighted spin-echo-sequence (TR: 600ms /TE:15 ms, scan time: 45 sec). The field of view (FOV) was 200 mm $\times$ 200 mm, using an 256 $\times$ 256 imaging matrix and two acquisitions. Slice thickness was 2 mm resulting in a pixel size of 0.79 mm $\times$ 0.78 mm. T1 and T2 relaxation-times within the pellets of the three tubes (CNN-Gd$^{3+}$, CNRN-Gd$^{3+}$, Magnevist) were measured to evaluate the intracellular relaxivity of the respective contrast agents (R=1/T1). The T1-relaxation time was measured by means of an inversion-recovery-sequence (TR: 5000 ms/ TE: 76 ms/TI: 25 - 4000 ms, 17 different TI-values, scan-time 15 $\times$ 25 sec, FOV: 160 mm $\times$ 160 mm, Matrix: 132 $\times$ 256, slice thickness: 7 mm, pixel size: 1,21 $\times$ 0.63 mm). T2 relaxation-time was measured by a multi-echo-sequence (TR: 5000 ms/ 16 TE-values: 30 ms - 245 ms, FOV: 250 mm $\times$ 250 mm, Matrix: 256 $\times$ 256, slice thickness: 5 mm, pixel size: 0.98 $\times$ 0.98 mm, scan time: 21 min 21 sec). Signal-intensity measurements were obtained from DU-145 carcinoma cells and background. A tube with DU-145 cells, incubated in MEM without contrast agent, was used as a control. In this way, the DU-145 cells were tested for uptake of the Gd$^{3+}$-complex-transporter when bound to either a NLS-sequence (#1552a) or a random-sequence (#1552b). As a control, the same procedure was performed in non-tumor cells (lymphocytes). Due to a signal intensity maximum in prostate cancer cells and lymphocytes after 3 hour's incubation, efflux measurements were begun after this time period. For this, both cell types were washed with conjugate-free MEM in order to remove all Gd$^{3+}$-complexes. This procedure was repeated hourly until no signal increase compared to the control tube (DU-145 prostate cancer cells or lymphocytes in MEM without contrast agent) could be detected in T1 weighted sequences. All experimental sequences were performed three times.

**Example 2**

**CNN-Gd$^{3+}$ enables cell nucleus molecular imaging of DU-145 prostate cancer cells**

The predominant aim of this study was to deliver the Gd-complex into the cell nucleus. This had been achieved previously using the plasma-membrane-translocation-peptide HIV-1 tat. This viral protein possesses nuclear-import characteristics. However, the HIV-1 tat peptide possesses not only a transactivating effect on the LTR (Long Terminal Repeat)-promoter but also can induce apoptosis in hippocampal neurons. As a consequence of these transactivating effects of HIV-1 tat peptide, a different method was chosen and human transport-peptide-units which show a comparable transport efficiency were examined (Table 1, #1552).

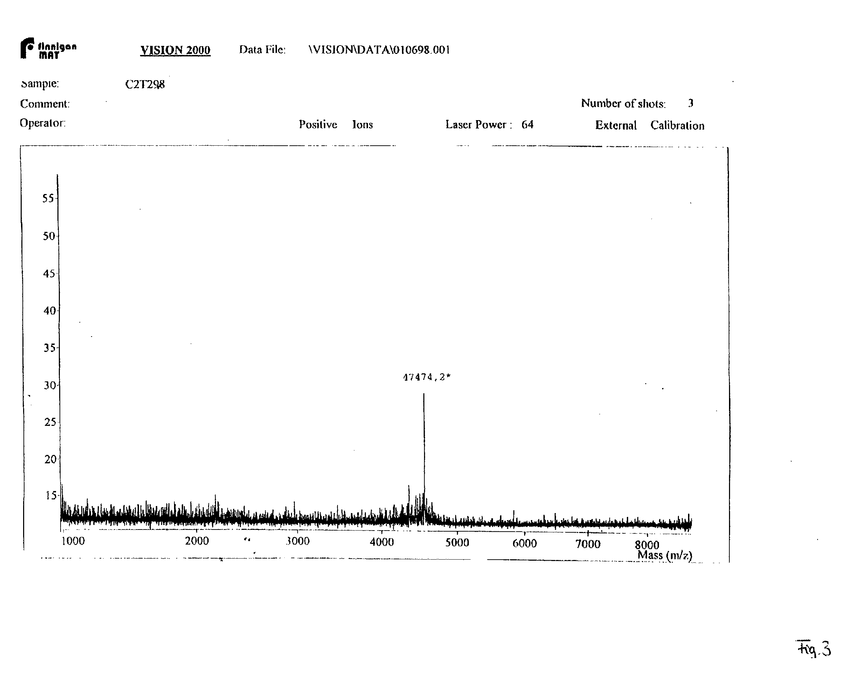

In parallel, molecular modeling was used to obtain the most appropriate spatial visualization of the conjugate (Figure 3). Although it is clear that the presented spatial structures of the bioconjugate are one approximate configuration of the many which flexible molecules such as peptides may exhibit, they represent to some extent a realistic spatial model: an all atom model of the complete molecules is presented, the shapes of the component modules are as have been reported for homologous structures, the relative sizes of the modules are correct and the physicochemical characteristics of the surface are represented.

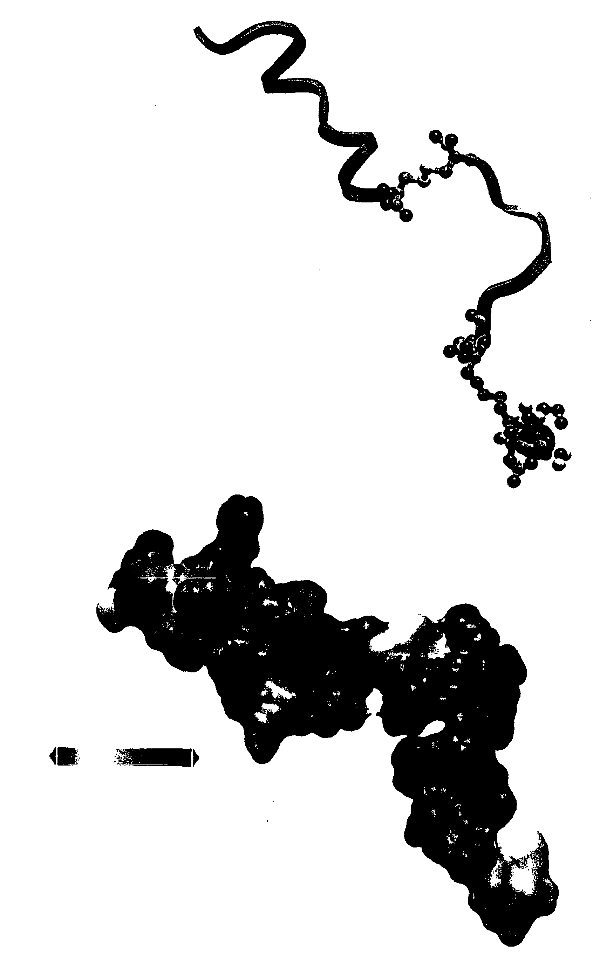

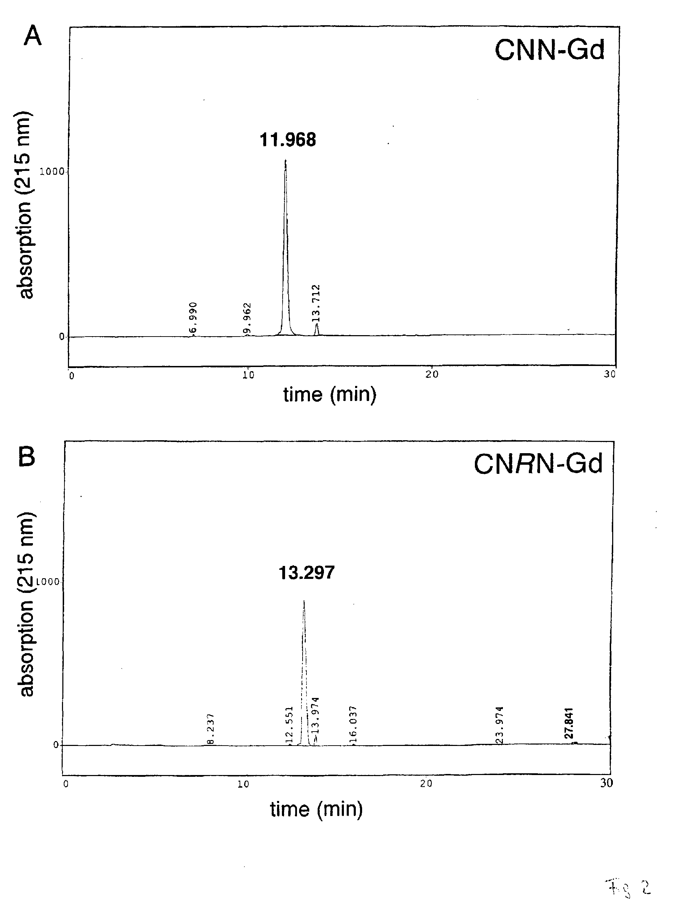

In MRI, an increased intracellular signal intensity in DU-145 cells could be detected after just 10 minutes incubation with the nuclear Gd-delivery system CNN-Gd$^{3+}$-complex (Figure 1) (Table 1, #1552a) (whole body 1.5 T Siemens Magnetom, standard circular polarized head coil). Due to the inability of MRI to recognize different cellular compartments, Confocal Laser Scanning Microscopy (CLSM) was further used to confirm the nuclear localization of the Gd$^{3+}$-complex. Due to the higher sensitivity of CLSM compared to MRI, lower concentrations of the CNN$^{FITC}$-Gd$^{3+}$-complex (Table 1, #1552af) could be used in CLSM (CLSM: 100 pm, MRI: 0.5 mM). In CLSM, a nuclear fluorescence signal was detected which would suggest that the CNN-Gd$^{3+}$-complex accumulated mainly at this site (Table 1, #1552a) (Figure 2 a). If, by way of comparison, Magnevist® alone was used as a contrast agent in MRI, there was no signal change above that of the DU-145 cells that had been incubated solely in Minimal Essential Medium (MEM). The measured relaxivity R within the DU-145-cell pellets changed by more than a factor of 5 after incubation with the Gd$^{3+}$-complex transporter (R=0.00354) as compared to that after incubation solely with Magnevist® (R=0.00069). The mass spectrometrically measured higher concentration of Gd$^{3+}$ within the nucleus compared to that in the cytoplasm (factorial difference: 40.000) could be explained by the interaction with Ran-GDP and importins. To show the nuclear specificity of the CNN-Gd$^{3+}$-complex, a CN*R*N-Gd$^{3+}$-complex (random-NLS) (Table 1, #1552b) was used resulting in a slightly higher MRI signal enhancement after 3 hours compared to that after using the specific NLS.

After the signal-intensity had reached its maximum after 3 hours, only a slight decrease was then observed over the next 45 hours when the CNN-Gd$^{3+}$-Complex was used (Figure 1). A possible explanation could be the lack of efflux of the CNN-Gd$^{3+}$-complex out of the nucleus (Table 1, #1552a). In contrast, the CN*R*N-Gd$^{3+}$-complex (Table 1, #1552b) could not enter the nuclear space and remained in the cytoplasm (Figure 2 b). The random sequence did not represent a suitable substrate for karyophyllic proteins (importins). Therefore, efflux was possible and a complete reduction of signal enhancement could be observed after just 8 hours (Figure 1). In CLSM, dual staining of both the $^{ALEXA}$CN*R*N$^{FITC}$-Gd$^{3+}$-complex {Gd$^{3+}$[DTPH]$_4$-HN-K$^{FITC}$-K-*Random*-C-S∩S-C-TPU$^{ALEXA}$} and $^{ALEXA}$CNN$^{FITC}$-Gd-complex {Gd$^{3+}$[DTPH]$_4$-HN-K$^{FITC}$-K-*NLS*-C-S∩S-C-TPU$^{ALEXA}$} was performed to determine whether ALEXA® 546 Fluor and FITC fluorescence signals were localized in the cytoplasm in the case of the random sequence or in the nucleus after using the specific NLS sequence (Table 1). By this method, the exact cytoplasmic localization of the Gd$^{3+}$[DTPH]$_4$-HN-K$^{FITC}$-K-*Random*-C-S∩S-C-TPU$^{ALEXA}$ (Table 1, #1552bf) could be confirmed (Figure 2 b) revealing the real source of the MRI signal enhancement. This could be explained by the fact that Gd$^{3+}$[DTPH]$_4$-HN-K$^{FITC}$-K-*Random*-C-S∩S-C-TPU$^{ALEXA}$ harboring the scrambled nuclear localization sequence (random) as well as the ALEXA®546 Fluor labeled TPU (Table 1, #1552bf) could not enter the cell nucleus and remained outside within the cytoplasm resulting in a mixed fluorescence signal (ALEXA®546 Fluor - red and FITC - green) (Figure 2 b).

In contrast, the Gd$^{3+}$[DTPH]$_4$-HN-K$^{FITC}$-K-*NLS*-C-S∩S-C-TPU$^{ALEXA}$ (Table 1, #1552af) was proven to be located within the nucleus (Figure 2 a). Some cells in Figure 2 a can be seen not to have taken gadolinium up into the nucleus which could be explained as follows: The transport of CNN-Gd$^{3+}$ into the nucleus is an active Ran-GDP dependent process and will not take place in cells functionally damaged during preparation but still apparently morphologically intact. However, the transport from the extracellular space into the cytoplasm is a passive process and would continue to take place even in functionally damaged cells. Additionally, some asynchronicity between cells with respect to the rates of nuclear uptake can be assumed. A slightly mixed fluorescence signal was also detected in the cytoplasm, whereas additionally a distinct sole green fluorescence signal (FITC) was observed within the nucleus (Figure 2 a). The cytoplasmic cleavage of the disulfide bond between the two modules ALEXA® 546 Fluor-tagged-TPU and the FITC-tagged NLS- Gd$^{3+}$-complex is followed by the effective nuclear import of the NLS-Gd$^{3+}$-complex$^{FITC}$ (Table 1, #1552af) (Figure 2 a). No evidence of cytotoxicity was observed after incubation with CNN- or CN*R*N-Gd$^{3+}$-complexes for 72 hours.

**Example 3**

**CNN-Gd$^{3+}$for chemo- and radiotherapy**

It is also conceivable that the CNN-Gd$^{3+}$-complex could simultaneously take on a diagnostic as well as a therapeutic role. To perform a highly efficient chemo- and radiotherapy it is indispensable to know the DNA repair enzyme activity in tumors. It would be decisive to determine such enzyme activity levels in MRI before further treatment was commenced.

A contrast agent called EgadMe (Louie et al., Nat. Biotech. *18*: 321-325, 2000) was applied by microinjection into the cell nucleus in order to measure galactosidase activity. Water access to the first or 9$^{th}$ coordination sphere of Gd was blocked with a substrate (e.g. galactopyranose) that could be removed by enzymatic cleavage. Following cleavage, Gd$^{3+}$ can interact directly with water protons to increase the MR signal. Galactopyranose was used as a blocking group which in turn enabled the measurement of the activity of galactosidase. A similar method can be used to visualize alkyltransferase, decisive for the outcome of chemotherapy (Louie et al., Nat. Biotech. *18*: 321-325, 2000), in the nucleus by first preventing water access to the first or 9$^{th}$ coordination sphere of a Gd$^{3+}$-complex with a suitable substrate which when enzymatically cleaved would lead to water access and a resulting increase in signal intensity in MRI.

**Claims**

1. A conjugate comprising (a) an amphiphilic transport peptide of human origin as a transmembrane module (TPU), (b) a nuclear localization sequence (NLS) and (c) a signalling and/or drug carrying module (SM).

2. The conjugate of claim 1, wherein the signalling and/or drug carrying module comprises Gd, Ga, Mn, I, Fe and/or F as (diagnostic) image creating compound.

3. The conjugate of claim 1 or 2, wherein the transmembrane module (TPU) is the human homeobox protein HOX-B1 or a fragment or derivative thereof having substantially the same biological activity.

4. The conjugate of claim 3, wherein the transmembrane module (TPU) comprises the amino acid sequence TQVKIWFQNRRMKQKK.

5. The conjugate according to any one of claims 1 to 4, wherein the nuclear localization sequence (NLS) comprises the amino acid sequence PKKKRKV or KPKRVKK.

6. The conjugate according to any one of claims 1 to 5, wherein the transmembrane module (TPU) is coupled to the nuclear localization sequence (NLS) via a covalently cleavable spacer I and/or the nuclear localization sequence (NLS) is coupled to the signalling and/or drug carrying module (SM) or a compound trapping the signalling and/or drug carrying module (SM) via a non-cleavable spacer II.

7. The conjugate according to claim 6, wherein spacer I comprises a cleavable disulfide bridge.

8. The conjugate according to claim 7, wherein spacer II is polylysine.

9. The conjugate according to any one of claims 6 to 8, wherein spacer II carries an FITC label.

10. The conjugate according to any one of claims 1 to 9, wherein the conjugate has the following structure: transmembrane module (TPU) - spacer I - nuclear localization sequence (NLS) - spacer II - signalling and/or drug carrying module (SM) or compound trapping the signalling and/or drug carrying module + signalling and/or drug carrying module (SM).

11. The conjugate of any one of claims 1 to 10, wherein said conjugate further comprises a cytotoxic drug.

12. Use of the conjugate of any one of claims 1 to 10 for the preparation of a diagnostic composition for cell tracking.

13. Use of the conjugate of any one of claims 1 to 10 for the preparation of a contrast agent for MRI.

14. Use of the conjugate of any one of claims 1 to 10 for the preparation of a diagnostic composition for determining the activity of DNA repair enzymes.

15. Use of the conjugate of any one of claims 1 to 11 for the preparation of a pharmaceutical composition for the chemotherapeutical treatment of a tumor.

16. Use of the conjugate of any one of claims 1 to 11 for the preparation of a pharmaceutical composition for the intranuclear GNCT-treatment of a tumor.

Fig 2

finnigan
mAT

**VISION 2000**   Data File:   \VISION\DATA\010698.001

Sample:   C2T298

Comment:   Number of shots:   3

Operator:   Positive   Ions   Laser Power :  64   External   Calibration

47474,2*

1000   2000   3000   4000   5000   6000   7000   8000
Mass (m/z)

Fig.3

*Fig 4*

a

b

Fig 5

**European Patent Office**

## EUROPEAN SEARCH REPORT

Application Number

EP 02 02 6700

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | WO 95 34295 A (UNIV VANDERBILT) 21 December 1995 (1995-12-21) * page 6, second paragraph; page 11, first paragraph; claims 11-15 * --- | 1-3,5-16 | C07K14/47 A61K49/14 A61K47/48 A61P35/00 |
| Y | HEIJNE VON G ET AL: "SPECIES-SPECIFIC VARIATION IN SIGNAL PEPTIDE DESIGN IMPLICATIONS FOR PROTEIN SECRETION IN FOREIGN HOSTS" FEBS LETTERS, ELSEVIER SCIENCE PUBLISHERS, AMSTERDAM, NL, vol. 244, no. 2, February 1989 (1989-02), pages 439-446, XP001149430 ISSN: 0014-5793 * page 439, left-hand column, second paragraph; page 439, right-hand column, last paragraph * --- | 1-3,5-16 | |
| Y | BRAUN K ET AL: "A biological transporter for the delivery of peptide nucleic acids (PNAs) to the nuclear compartment of living cells" JOURNAL OF MOLECULAR BIOLOGY, LONDON, GB, vol. 318, no. 2, 2002, pages 237-243, XP002238940 ISSN: 0022-2836 * abstract; table 1; page 242, left-hand columns, lines 5-9 * --- -/-- | 5-10 | TECHNICAL FIELDS SEARCHED (Int.Cl.7) C07K A61K |

The present search report has been drawn up for all claims

| Place of search | Date of completion of the search | Examiner |
|---|---|---|
| MUNICH | 26 May 2003 | Fausti, S |

EPO FORM 1503 03.82 (P04C01)

European Patent Office

**EUROPEAN SEARCH REPORT**

Application Number

EP 02 02 6700

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | BHORADE RAJEEV ET AL: "Macrocyclic chelators with paramagnetic cations are internalized into mammalian cells via a HIV-tat derived membrane translocation peptide." BIOCONJUGATE CHEMISTRY, vol. 11, no. 3, May 2000 (2000-05), pages 301-305, XP002242549 ISSN: 1043-1802 * abstract; page 301, right-hand column, lines 5-8; page 302, left-hand column, lines 1-4; figure 3 * | 2,12-14 | |

TECHNICAL FIELDS SEARCHED (Int.Cl.7)

The present search report has been drawn up for all claims

| Place of search | Date of completion of the search | Examiner |
|---|---|---|
| MUNICH | 26 May 2003 | Fausti, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

24

## ANNEX TO THE EUROPEAN SEARCH REPORT
## ON EUROPEAN PATENT APPLICATION NO.

EP 02 02 6700

This annex lists the patent family members relating to the patent documents cited in the above-mentioned European search report.
The members are as contained in the European Patent Office EDP file on
The European Patent Office is in no way liable for these particulars which are merely given for the purpose of information.

26-05-2003

| Patent document cited in search report | Publication date | Patent family member(s) | Publication date |
|---|---|---|---|
| WO 9534295        A | 21-12-1995 | US    5807746 A<br>AU   2828095 A<br>WO   9534295 A1<br>US    6043339 A<br>US    6495518 B1 | 15-09-1998<br>05-01-1996<br>21-12-1995<br>28-03-2000<br>17-12-2002 |